# EUROPEAN PATENT APPLICATION

(11) **EP 1 872 732 A1**
(43) Date of publication of application: **02.01.2008**
(21) Application number: 06741694.1
(22) Date of filing: 20.04.2006
(51) Int. Cl.: A61B 17/80

(54) **BONE PLATE WITH CENTRIC HOOKS**

(30) Priority: 21.04.2005 CN 200510025297
(71) Applicant: Zhang, Rongkui, Jiangsu 215400 (CN); Xia, Yuanhai, Jiangsu 215400 (CN)
(72) Inventor: Zhang, Rongkui, Jiangsu 215400 (CN); Xia, Yuanhai, Jiangsu 215400 (CN)
(74) Representative: Metman, Karel Johannes
(86) International application number: PCT/CN2006/000747
(87) International publication number: WO 2006/111096

(57) **Abstract**

This invention provides a bone plate for treating bone fractures comprising a base plate (1) with an arc or arch cross section, and hook-shaped angular reinforcements (2) which have blades thereon provided on the two ends of the concave surface for contacting bones of the base plate (1). At least one tooth (3) is arranged longitudinally on the blades of the angular reinforcements at intervals. The hook-shaped angular reinforcements at the two ends of the cross section present to be centripetal hooks at the engagement points of the blades and the periosteum of fractured bone. The base plate (1) is provided with thread holes (4), installing holes (6) for pressing screws, and adjusting holes (5) for aligning and restoring fractured bones. An adjusting setscrew (7) may be provided in the corresponding hole of the plate.

## Description

### Field of The Invention

This invention relates to a bone plate that used for bonesetting to treat fractures, especially a bone plate with centripetal hooks.

### Background of The Invention

In the prior art, the traditional A0-series bone plate is widely used in treating bone fractures at any part of the body. The bone plate has a glossy surface used to contact the bone, which would lead to a broad contact area between the bone plate and periosteum after the bone plate being fixed, consequently bringing the following blights as that:
1. Hominine bones and the bone plate form slidably and dissociatively contaction, for the surface of hominine bones are glossy as the traditional bone plates, bringing on difficulty for the bones locating when bone connecting operation is going on, and therefore strengthening torque of fixing setscrew being necessarily carried out, but too-large torque would potentially make setscrew disengage from the bone, and thereby setscrew drawn, setscrew rupture, plate bend and plate rupture would easily occur for the influence of the circumambient force;
2. Although it is stable to connect bones by the traditional bone plate in static condition, when the patient move to postoperative environment to bear the compositive forces, the stability at engagement point (being equivalent to end of fractured bone) can't be ensured because of the dissociating state between the plate surface and the bone surface, that might cause the bone engaged at changed location to be malformed;
3. This traditional bone plate will contact with bone body with pressure in large area, causing pressure-sheltering effect, resulting in that at the evening period of concrescence, the bone underneath the plate cannot get sufficient stimulation, so in order to achieve the aim of firm fixure and anatomic restoration, the blood supply of the bone would often badly damage, leading to osteoporosis and decline of power performance, and even causing re-fracture after the bone plate removed; and
4. Although the traditional bone plate is widely used because of its simple structure, low cost and easiness in manufacture, lots of the disadvantages mentioned above are the hidden troubles during fracture treatment all the while.

Referring to two published CN patents (with patent number of ZL95230870.3 and ZL03213488.6), each of them provided a vaulted steel plate with tracking tooth used for treating fractures, making improvements to the traditional bone plate, but the vaulted steel plate still have some shortcomings on structure as following enumerated.
1. The main character of each bone plate is that it comprises a vaulted base plate and two tracks symmetrically set at the two sides, with quadrate or round tooth thereon. Consequently tracks and tooth superposed to be taper and/or quadrate in structure, causing too many edges and corners at the step interface for the tooth to be conveniently processed. Especially for conic tooth, the process art at the track surface would be rather complex.
2. Each bone plate has vaulted base plate with track-girders symmetrically set thereon, which restricts shape of bone area according to frequently anatomic bone, but for special, anomalous and/or unsymmetrical bodies, the track-girders with teeth set thereon which increase the height of the whole bone plate, may cause hominine skin bulgy after operation.
3. The teeth peaks and track surface of this bone plate form steps, the surface line of the track and the inner vaulted-chimb line of the bone plate form pointedness. Because of bone plate with such geometric structure and tubular circumferential bone body, in the process of bone-connection operation, the bone plate bestrides on the bone body. If the bestriding distance is too large, the inner bias of the teeth are tangential to the longitude of the bone body by any possibility, that will cause the pointedness that formed by the track and the inner vaulted-chimb in contact with the bone body, resulting in the teeth losing function of bitten into the bone skin; and the pointedness which is a longitudinal line impacting the bone body, causing shelter effect to prevent blood latitudinal transportation the same as regeneration. But if decrease the bestriding distance of the bone plate to achieve contact between the bone body and the teeth at points, that will make bone plate lose round force and decrease strength of anti-cut, anti-bend and anti-torsion, the inner side line of conic teeth at the two ends of the bone plate will lose its centripetal force when it bear the force, for it will spread along the tangential direction to the outside diameter of the bone body.

### Summary of The Invention

The present invention generally relates to a bone plate with centripetal hooks which may fit every kind of anatomic form of bone fractures, have simple structure and good function, and meet the treating requirements and stable therapy effect, to overcome the shortcomings of the bone plate according to the prior art.

The target present invention can be realized as following:
A bone plate for treating bone fractures comprises a base plate with an arc or arch cross section; hook-shaped angular reinforcements having blades thereon, provided on the two ends of the concave surface for contacting bones of the base plate or at periphery of the bone plate, and presenting to be centripetal hooks on the two ends of the cross section of the base plate at the occlusion places between the blades and periosteum; and at least one tooth arranged longitudinally on the blades of the angular reinforcements at intervals.
The base plate with arc or arch cross section make such bone plate have greater capability of anti-curve, anti-fold, anti-torsion, anti-rotate, anti-tire than the traditional rectangular bone plate. The geometric structure of hook-shaped angular reinforcements being located on the two ends of the base plate, enhances the strength and the stiffness for the arc or arch base plate, to endure required combined stress at the fracture part and torsional stress produced by torsion load, and also enhances anti-bend and anti-torsion strength of the bone plate needed for fixing bone.
The centripetal hooks at the two sides of the bone plate have spires bestriding on the bone body, which will bite into the bone surface under pressure to enhance ability of anti-move, anti-rotate and anti-cut for the bone plate. Under the action of the combined stress around the bone, the centripetal spires of the teeth at the two sides of the bone plate form jaw-shaped towards the center of the bone, and buckle with corresponding each other to prevent the looseness of the bone plate and further prevent the setscrew being drawn, folded or broken and the plate being bent or broken. So generally this firmly fixing bone plate need not be additionally fastened outside after operation.
The teeth contact bone cortex forming interspace between the surface of the bone plate and the bone surface, that makes large contact area between the bone plate and the bone cortex be avoided, and ensures blood regeneration and transportation at the fracture end as well as around the bone cortex, resulting in reducing shelter effect and accelerating the generation of scab and concrescence of fracture, in order to make patient do some exercise at the forepart. The interspace between the bone plate and the bone also can be used to let a steel wire with tensility pass through, being propitious to enlacement and relocation for treating smashing fracture.
The said base plate is provided with thread holes, installing holes for pressing screws, and adjusting holes for aligning and restoring fracture bones. An adjusting setscrew may be provided in the corresponding thread hole of the plate. That is for special steel bone plate having equidimension of bestriding distance, for the adjusting setscrew at the corresponding position can form interlaced occlude with the hook-shaped teeth at the two sides, so as to ensure the interspace between the bone plate and the bone surface as well as multidimensional fixing for the bone plate, and also react on smashing fracture.
The centripetal hook-shaped teeth and the adjusting setscrew share the load, that avoiding concentration of stress and reducing bolts for fixing longer bone plate, to mitigate damage to blood supply and osseous damage caused by large numbers of bolts.
The two edges of the base plate and the bone center form fan-shaped, wherein the edge lines at the intersection near bone skin bending towards the inner arc directly, to form hook-shaped angular reinforcements.
The spires of the teeth and the blade of the angular reinforcements are compactly concerted, lowering the height of the whole bone plate. The setscrew neatly and conveniently matches with the bone plate, making the whole structure be easily molded.
It is, therefore, an advantage of the present invention to provide a bone plate which is beseemed to be manufactured with any standard of shape according to skeletal anatomic shape and the bone structure.
Additional features and advantages of the present invention are described in, and will be apparent from, the detailed description of the present embodiment and from the drawings.

### Brief Description of The Drawings

Fig.1 is a sketch map indicating structure of the target invention;
Fig.2 is the A-A section view of Fig.1;
Fig.3 is the top view of Fig.1;
Fig.4 is a section view of the target invention in use showing the technical principle of the invention;
Fig. 5 is a section view of an arc base plate with centripetal hooks;
Fig.6 is a section view of a curving base plate with centripetal hooks.

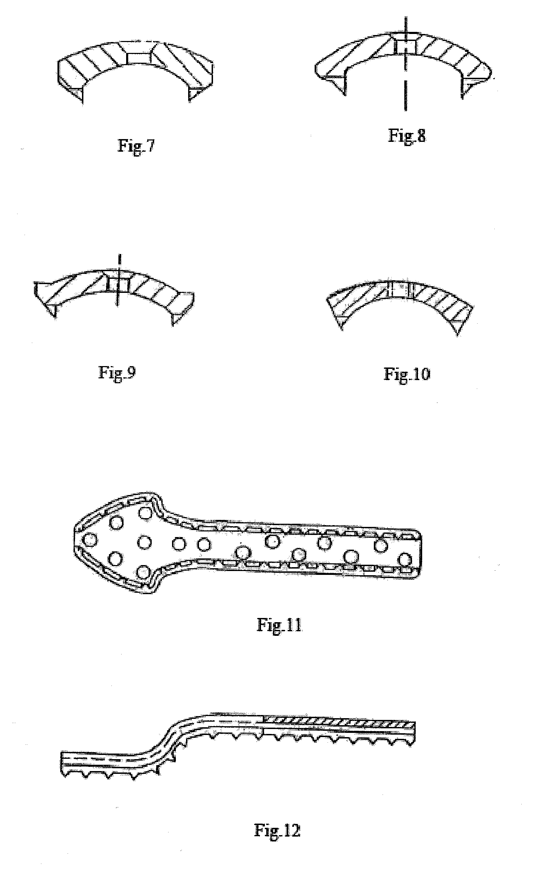

Fig.7 is a section view of an arch base plate with centripetal hooks;
Fig. 8 is a section view of an arc base plate with centripetal crooked hooks;
Fig.9 is a section view of a wing-edged arc base plate with centripetal hooks;
Fig. 10 is a section view of an arch base plate with intrados directly connected to centripetal hooks;
Fig.11 is a sketch map of another bone plate with specific edge;
Fig.12 is the top view of the Fig.11;

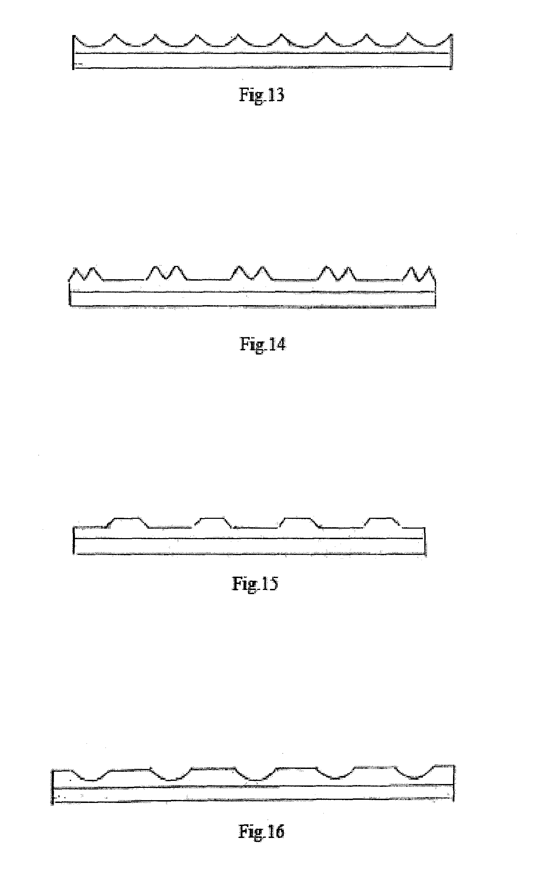

Fig.13 is a sketch map of a bone plate with tined-blade teeth that arranged longitudinal;
Fig.14 is a sketch map of another bone plate with tined-blade teeth that arranged longitudinal;
Fig.15 is a sketch map of a bone plate with plane-blade teeth that arranged longitudinal; and
Fig.16 is a sketch map of another bone plate with plane-blade teeth that arranged longitudinal;
Wherein: 1. base plate; 2. hook-shaped angular reinforcements; 3. tooth; 4. thread holes; 5. adjusting holes; 6. installing holes; 7. adjusting setscrew;

### Detailed Description of A Preferred Embodiment

The present invention relates to a bone plate, which would be further explained as following with the above-mentioned drawings and a preferred embodiment.

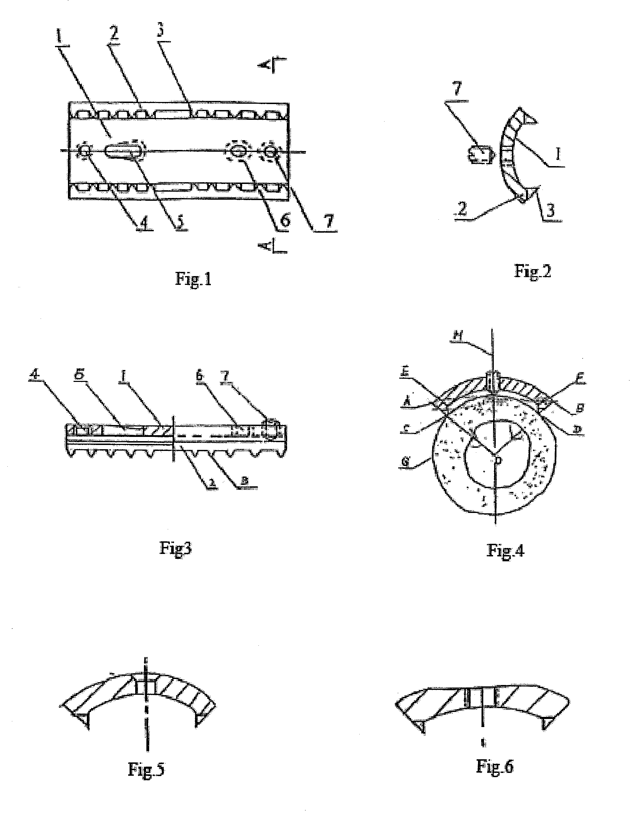

Referring to Fig.1 to Fig.3, a bone plate with centripetal hooks comprises a base plate 1, and hook-shaped angular reinforcements 2 which have tined blades thereon, the tined blades having teeth 3 longitudinally arranged on. The said base plate 1 is provided with thread holes 4, adjusting holes 5 for aligning and restoring fracture bones, installing holes 6 for pressing screws and adjusting setscrews 7.

The hook-shaped angular reinforcements 2 located at the two ends of the concave surface for contacting bones or the profile edge of the base plate, at least one tooth 3 on the angular reinforcement 2 is arranged longitudinally on the blades of the angular reinforcements at intervals.

Referring to Fig.4, A and B represent the two endpoints of outer arc line of the base plate 1, E and F represent the two endpoints of inner arc line of the base plate 1, C and D represent the two occlusion points between the blade spires and the bone skin; G represents the skin line of the outside diameter of the bone; O represents the navel of the bone circle, and H represents the central line passing through the point O.

The technical principle should be described as following.

Set the thickness of the bone plate between the out arc line AB and the inner arc line EF; set the gap between the inner arc line EF and the bone skin G; set the outside diameter of the bone being based on confirming the navel O; and make a beeline H from the navel O via the outside diameter of the bone. Symmetrically set the length of outer arc line AB on two sides of beeline H, and set the length of the inner arc line EF as also. Set a link line from the endpoint A to point O or a set point on the beeline H, which meets the outside longitude of the bone at point C, the same as setting a link line BO meeting the outside longitude at point D. Then link the endpoint E with point C and the endpoint F with point D. In other words, use C, D as blade spires forming two hook-shaped angular reinforcements of angle ACE and angle BDF. So the hook-shaped angular reinforcements 2 presents to be centripetal teeth on the two ends of the cross section at the occlusion point between its blade spires and the bone skin.

Under pressure, cut tension and torsional tension, the combined tension pass along the line AO and BO to the navel O. Points C and D are the force-shared points, the angle ACE and the angle BDF formed from point C and point D mutually forming the triangle support structure, by which multidimensional forces should pass through outside diameter of the bone plate, to achieve a balance of combined forces. To meet the requirements of fixing biological-mechanics and biological fixure, for the scarfskin with limited fracture area, the limited interspace between the bone plate and the bone skin, the limited thickness and height of the bone plate, and to meet the requirement of mechanics for the bone plate, the bone plate with centripetal hooks provided by the present invention will be an excellent technical scheme, and also be the essentially differentiate in structure and function with the prior art.

The method for use of the bone plate according to the present invention can be described as following in details that:
The use of the present bone plate is similar to traditional bone plates, but an attention to use of adjusting setscrew, for first pre-tightening the setscrew with pressure after the teeth arranged around the edge of the bone plate contacting the bone skin, secondly adjusting the setscrew until its spire in contact with the bone skin, then properly knocking the bone plate to make the teeth tines all tightly contact the bone skin, and finally tightening screws near the fracture end to the screws away from the fracture end alternately and ordinally, to achieve firmness of connection of bone and bone plate.

## Claims

1. A bone plate with centripetal hooks comprising a base plate (1) with an arc or arch cross section, is **characterized in that**:
Hook-shaped angular reinforcements (2), having blades thereon, and presenting to be centripetal hooks on the two ends of the cross section at the engagement points between the blades and periosteum, are provided on the two ends of the concave surface for contacting bones of the base plate (1) or at periphery of the bone plate; with
At least one tooth (3) arranged longitudinally on the blades of the angular reinforcements (2) at intervals.

2. The bone plate with centripetal hooks as claimed in claim 1 wherein the said base plate (1) is provided with thread holes(4), installing holes (6) for pressing screws, and adjusting holes (5) for aligning and restoring fractured bones.

3. The bone plate with centripetal hooks as claimed in claim 1 wherein an adjustable setscrew (7) may be provided in the corresponding thread hole of the plate.
